# EUROPÄISCHE PATENTSCHRIFT

(11) **EP 2 114 325 B1**
(45) Veröffentlichungstag und Bekanntmachung des Hinweises auf die Patenterteilung: **22.08.2012**
(21) Anmeldenummer: 08717284.7
(22) Anmeldetag: 29.02.2008
(51) Int. Cl.: A61F 5/055

(54) **VORRICHTUNG ZUM ENTLASTEN UND THERAPIEREN DER CERVICALEN WIRBELSÄULE**
DEVICE FOR THE DECOMPRESSION AND THERAPY OF THE CERVICAL SPINAL COLUMN
DISPOSITIF POUR SOULAGER ET SOIGNER LA COLONNE VERTÉBRALE CERVICALE

(30) Priorität: 02.03.2007 CH 348072007
(43) Veröffentlichungstag der Anmeldung: 11.11.2009
(73) Patentinhaber: CuraCeres GmbH, 6072 Sachseln (CH)
(72) Erfinder: Fischer, Paul Walter, 11400 Fonters du Razes (FR)
(74) Vertreter: Felber, Josef
(86) Internationale Anmeldenummer: PCT/EP2008/052504
(87) Internationale Veröffentlichungsnummer: WO 2008/107392

(56) Entgegenhaltungen:
- WO-A-98/43568
- WO-A-03/079941
- WO-A-2006/102602
- DE-U1- 29 613 213
- US-B1- 6 447 468

## Beschreibung

Die vorliegende Erfindung betrifft eine Vorrichtung, um die Wirbelsäule im cervicalen Bereich zu entlasten und sie regelmässig zu therapieren. Viele Menschen leiden an Kopfschmerzen, die oftmals von der Wirbelsäule ausgelöst werden, vor allem vom Nackenbereich der Wirbelsäule, also dem cervicalen Bereich. Weiter gibt es viele Menschen, die Schleudertramatas erlitten haben und mit chronischen Schmerzen im Nackenbereich zu kämpfen haben. Die Wirbelsäule im Nackenbereich muss ständig das Gewicht des Kopfes tragen und alle Momente aufnehmen und absorbieren, die von Kopfbewegungen ausgehen. Entsprechend ist sie belastet und bei Schädigungen infolge Unfällen oder Abnützungen überaus empfindlich. Aus dem Stand der Technik sind mehrere Konstruktionen bekannt, welche jedoch hauptsächlich Halsstützen sind. So offenbart etwa die DE 296 13 213 U1 der als nächter Stand der Technik anerkannt wird, eine medizinische Halskrawatte mit einer Anzahl Luftkammern zum dosierten Aufblasen. Es handelt sich um ein medizinisches Gerät, um bei Verletzungen und Beschwerden im Halswirbelsäulenbereich als Stütze verwendet zu werden, jedoch nicht um ein Gerät zum Therapieren. Es ist nicht möglich, den Kopf gewünschten Richtung zu neigen, durch Strecken der Halswirbelsäule. WO03/079941 A zeigt eine rein mechanische Lösung zum Strecken des Halses. Der Kopf wird dabei aber nicht gleichzeitig um eine Schwenkachse nahe des Kinns nach vorne geschwenkt. Vielmehr wird er gerade vom Torso nach oben gespannt, und zwar mittels Schrauben, angetrieben über Winkelgetriebe. Dadurch ist es nicht möglich, bei Auftreten von plötzlichen Schmerzen eine rasche Entlastung herbeizuführen. US 6 447 468 zeigt ein ähnliches Gerät wie hier vorgeschlagen, bei welchem aber die wichtige Abstützung des Kopfes auf seiner Vorderseite fehlt. Um eine wirksame Therapierung der Wirbelsäule zu erreichen, müssen alle Halsmuskeln völlig entspannt sein und der Drehpunkt des Kopfes beim Anheben durch das Gerät sollte vorne in der Nähe der Kinnspitze liegen. WO98/43568 zeigt ein Gerät, welches nicht vom Patienten selbst angelegt und bedient werden kann. Es dient bloss zum stationären Stützen des Kopfes, nicht aber zum effektiven therapeutischen Beanspruchen der Halswirbelsäule auf Zug. Schliesslich zeigt W02006/102602 ein Gerät zum Strecken der Halswirbelsäule und auch der Rückenwirbelsäule. Das Gerät erlaubt viele Funktionen, ist aber aufgrund seiner Auslegung und aufwändigen Konstruktion nur für einen stationären Einsatz gedacht und kaum geeignet, überall hin mitgenommen zu werden und es eignet sich nicht, um rasch und einfach alleine von einer zu therapierenden Person selbst angelegt zu werden. Die bisher bekannt gewordenen Lösungen vermögen daher nicht in jeder Hinsicht zu befriedigen.

Bisher fehlt eine Vorrichtung und ein Gerät, mit dem ein mit solchen vom Nackenbereich der Wirbelsäule ausgelösten Schmerzen und Beschwerden belasteter Mensch selbst einfach und ambulant, bei sich zu Hause oder gar unterwegs auf Reisen seine Wirbelsäule im Nackenbereich einfach, gezielt und individuell einstellbar entlasten und auch therapieren kann. Die Physiotherapeuten bestätigen, dass eine Entlastung, und besonders periodisches Entlasten und Belasten dem Wohlbefinden und einem Heilprozess sehr förderlich sind. Dieser Sachverhalt wurde durch praktische Erfahrung bestätigt und erhärtet.

Die Aufgabe der vorliegenden Erfindung ist es deshalb, eine Vorrichtung zum Entlasten und Therapieren der cervicalen Wirbelsäule zu schaffen, welche einfach und ambulant, zu Hause oder gar unterwegs auf Reisen einsetzbar ist, und die eine gezielte, individuell einstellbare Entlastung der Wirbelsäule im Nackenbereich ermöglicht. Die Vorrichtung soll auch ein gezieltes Therapieren ermöglichen, nämlich eine periodisches Entlasten und wieder Belasten der Wirbelsäule im Nackenbereich.

Diese Aufgabe wird gelöst von einer Vorrichtung zum Entlasten und Therapieren der cervicalen Wirbelsäule nach dem Oberbegriff und mit den kennzeichnenden Merkmalen des Patentanspruches 1.

Diese Vorrichtung, ihre Bestandteile und ihr Aufbau werden anhand der nachfolgenden Zeichnungen im Einzelnen beschrieben und ihre Funktion wird erläutert und erklärt.
Es zeigt:
- Figur 1:: Die einzelnen Bestandteile einer Vorrichtung getrennt voneinander und übereinander angeordnet dargestellt;
- Figur 2:: Die Vorrichtung an eine zu therapierende Person angelegt, von der Seite her gesehen;
- Figur 3:: Die Vorrichtung an eine zu therapierende Person angelegt, von schräg hinten gesehen;
- Figur 4:: Die Vorrichtung an eine zu therapierende Person angelegt, von schräg vorne gesehen;
- Figur 5:: Eine Vorrichtung gemäß der Erfindung mit geöffneten Schenkeln von oben gesehen;
- Figur 6: Die Vorrichtung gemäß der Erfindung nach Figur 5 geschlossen in Anwendungslage gezeigt.

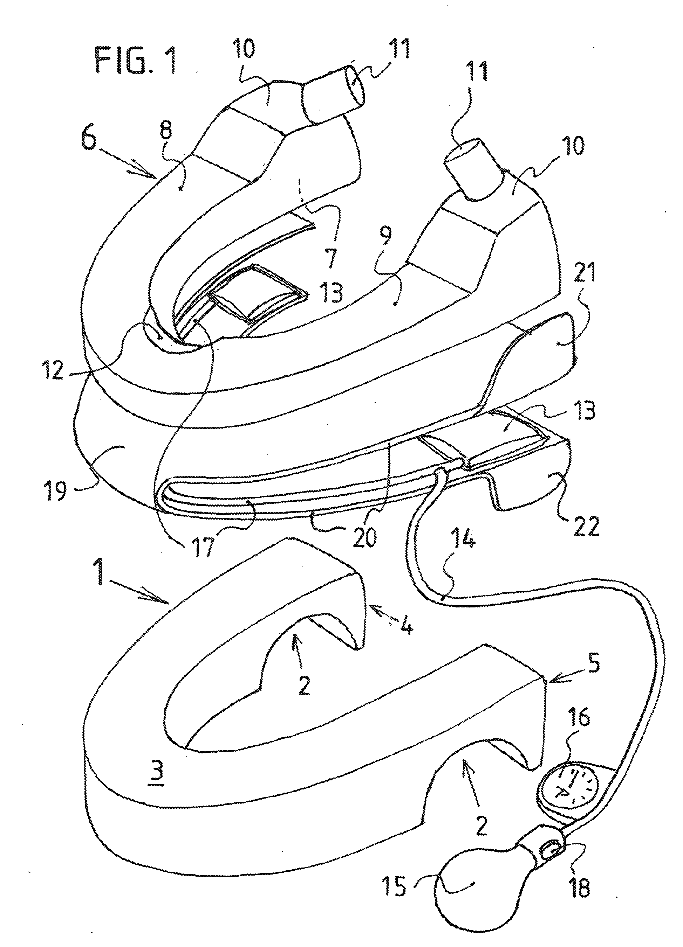

Die Figur 1 zeigt zunächst die einzelnen Bestandteile einer Vorrichtung. Diese besteht aus einem Schulter-Formstück 1 aus einem leichten, formelastischen Material, wie Polystyrol oder ähnlich. Polystyrol ist ja ein amorpher teilkristalliner Thermoplast. Bekannt ist dieses Material etwa unter den Handelsnamen Lustron, Styropor, Styrodur, Styroflex, Sagex (in der Schweiz) und Telgopor (in der spanischsprachigen Welt). Dieses Schulter-Formstück kann indessen auch aus einem alternativen Material mit ähnlicher Beschaffenheit gefertigt sein. Es kann etwa ein Holz- oder Sperrholz-Körper sein, oder ein geschäumtes Kunststoff-Material oder ein geblasener hohler Kunststoff-Körper kann in Frage kommen. Vorteilhaft ist der Formkörper von einem Textilstoff eingefasst, welcher auswechselbar ist, und daher zum Beispiel als formschlüssige Tasche ausgeführt ist, die mittels eines oder mehrer Reissverschlüsse, oder mittels Klettverschlüssen oder Druckknöpfen um den Formkörper verschliessbar ist. In einer anderen Variante kann der Formkörper auch mit einem geeigneten Material beschichtet sein, dass sich auf der Haut angenehm anfühlt, und das auch gut abwaschbar ist. Dieser Formkörper bildet die Form eines Hufeisens oder eines U's mit seinen zwei Schenkeln 4,5 und an deren hinterer unterer Seite ist eine kännelartige, quer zur Hufeisenform oder U-Form verlaufende Einbuchtung 2 ausgenommen. Mit dieser Einbuchtung 2 ist das Formstück 1 dazu bestimmt, auf der Schulter der zu therapierenden Person aufzuliegen. Auf der Oberseite 3 des Schulter-Formstücks 1 ist dieses eben ausgeführt. Zum Anlegen werden die beiden Schenkel 4,5 der Hufeisenform leicht elastisch auseinander gespreizt, sodass das Schulterformstück 1 von vorne her, den Hals der zu therapierenden Person umschliessend, auf deren Schultern geschoben werden kann und hernach mit der kännelartigen Einbuchtung 2 auf den beiden Schultern der Person aufliegt. Wenn der Formkörper allzu steif ist, kann an seinem vorderen Ende ein Scharnier mit vertikaler Schwenkachse eingebaut sein, sodass sich die beiden nach hinten ragenden Schenkel voneinander wegschwenken lassen. Dieses SchulterFormstück 1 ist einige Zentimeter dick ausgeführt, sodass trotz seiner Einbuchtung 2 auf seiner Unterseite durch die Oberseite eine ebene Auflagefläche gebildet wird. Die lichte Weite zwischen den beiden Schenkeln 4 und 5 ist so bemessen, dass mit den Schenkeln 4,5 die Hälse von allen Kragenweiten umschlossen werden können.

Das zweite, unabdingbare Bestandteil der Vorrichtung ist das Kopf-Formstück 6, welches ebenfalls von einer Textiltasche eingeschlossen sein kann oder mit einem Textilstoff eingefasst sein kann oder beschichtet sein kann. Dieses ist aus dem gleichen Material gefertigt wie das Schulter-Formstück und bildet auch die Form eines Hufeisens oder U's. Die Unterseite 7 dieses Kopf-Formstückes 6 ist eben ausgeführt, während es auf der Oberseite gegen die Hinterseite der beiden Schenkel 8,9 je eine Erhöhung 10 aufweist, auf deren inneren oberen Seite je ein Höcker 11 schiefwinklig gegen oben ragt. Vorne in der Oberseite des runden Abschnittes des Kopf-Formstückes 6 ist eine Ausnehmung 12 ausgeformt, in welche das Kinn einer zu therapierenden Person einpasst. Die Höcker 11 bilden ebenfalls Auflagepunkte, nämlich für den unteren Randabschluss des Schädels, links und rechts der cervicalen Wirbelsäule, sodass also eine Dreipunkt-Auflage des Kopfes bzw. Schädels mit dem Kinn auf der Oberseite des Kopf-Formstückes 6 erzielt wird. In einer Variante können diese Höcker 11 als gesonderte zylinderförmige Teile aus einem Gummi, aus Kunststoff, Holz oder Polystyrol hergestellt sein, und sie sind dann in entsprechende Löcher an den beiden Schenkeln des Formstückes 6 einsteckbar. Dadurch können verschieden hohe Höcker 11 eingesetzt werden, je nach den anatomischen Verhältnissen bei der zu therapierenden Person. Die Oberseite bzw. Auflagefläche der Höcker 11 können durch Zuschneiden noch besser an die Auflage am Schädel angepasst werden, sodass ein bequemer Pass-Sitz erreicht wird. Dieses Kopf-Formstück 6 kann auch so konstruiert sein, dass es im Wesentlichen aus einem U-förmig gebogenen Metallprofil besteht, welches an der Vorderseite eine Formstück-Polsterung für die Kinnauflage aufweist. Diese Kinnauflage kann am Profil mittels Schrauben höhenverstellbar befestigt sein, sodass die Kinnauflage individuell anpassbar ist. An den beiden Enden der Schenkel des U-förmigen Metallprofils sind verstellbare Abstützhöcker vorhanden, die über Schrauben in ihre Lage, ihrem Anstell-Winkel und in ihrer Höhe verstellbar sind. Im vorderen Bereich des Metallprofils kann ein Scharnier eingebaut sein, sodass die beiden Schenkel in ihrer Schwenklage verstellbar sind. Die Länge der beiden seitlich verlaufenden Profile bzw. Schenkel ist ebenfalls verstellbar, zum Beispiel, indem sich diese dort überlappen oder teleskopisch ineinander geführt sind und in jeder Auszugslage aneinander fixierbar sind. Damit kann die Länge des Kopf-Formstückes 6 als Ganzes sowie auch die Position und Höhe der Kinnauflage sowie dier beiden Höcker ganz individuell und exakt an jede Person angepasst werden.

Als weiteres unabdingbares Bestandteil gehört eine Hebeeinrichtung zur Vorrichtung, die zwischen den beiden aufeinanderliegenden Formstücken 1,6 wirkt und das Kopf-Formstück 6 vom unteren Schulter-Formstück 1 anhebbar macht. Am einfachsten und besten eignet sich eine pneumatisch wirkende Hebeeinrichtung. Sie besteht im gezeigten Beispiel aus zwei Luftkissen 13, die auf den beiden hinteren Seiten der Vorrichtung zwischen den Schenkeln 8,9 des oberen 6 und unteren Formstückes 1 eingelegt sind. Diese beiden Luftkissen 13 sind über einen Schlauch 17 miteinander verbunden und über einen weiteren Schlauch 14 aufpumpbar, indem an dessen Ende ein Gummibalg 15 montiert ist. Im Schlauch 14 ist ausserdem ein Entlastungs-Einwegventil 18 eingebaut, mit einem federbelasteten Druckknopf zur Öffnung des Ventils 18. Zusätzlich kann in den Schlauch 14 ein Manometer 16 eingebaut sein, zur Anzeige des in den Kissen 13 herrschenden Luftdruckes.

Im gezeigten Beispiel schliesst die Vorrichtung noch eine V-förmige Zwischenplatte 19 aus Kunststoff, Blech oder Sperrholz auf. Diese schliesst von der Seite her gesehen einen spitzen Winkel ein und ihre Aussenränder 20 verlaufen längs der Aussenkontur der beiden anderen Stücke, nämlich des Schulter- 1 und des Kopfstückes 6. Diese Zwischenplatte 19 weist an ihrem oberen Schenkel auf dessen hinterer Seite aussen je ein nach oben ragendes Ohr 21 auf, und gegebenenfalls auch am unteren Schenkel der Platte je ein nach unten ragendes Ohr 22, sodass das Kopf-Formstück 6 zwischen diesen beiden Ohren 21 gehalten oder zumindest geführt ist, und das Schulter-Formstück 1 in geleicher Weise zwischen den nach unten ragenden Ohren 22. Diese Vorrichtung wird nun so angelegt, dass zunächst das Schulterformstück 1 von vorne auf die Schultern einer zu therapierenden Person aufgeschoben wird. Selbstverständlich kann diese Person dieses Formstück 1 wie auch die ganze Vorrichtung ohne Weiteres selbst anlegen. Wenn das Schulter-Formstück 1 angelegt ist, wird als Nächstes das Kopf-Formstück 6 ebenfalls von vorne auf die ebene Oberseite des Schulter-Formstückes 1 aufgeschoben. Hierzu müssen die beiden Schenkel 8,9 wiederum leicht voneinander weggespreizt werden und hernach durch Loslassen wieder elastisch geschlossen werden. Jetzt liegt das Kinn der Person auf der Ausnehmung 12 am Kopf-Formstück 6 auf, und die beiden Höcker 11 an der hinteren Oberseite der Schenkel 8,9 stützen den unteren Rand des Schädels auf seiner hinteren Seite. Dadurch ist eine saubere Dreipunkt-Auflage gebildet. Als Nächstes wird die Zwischenplatte 19, die von oben gesehen eine ähnliche Hufeisenform aufweist, von vorne zwischen Schulter-Formstück 1 und Kopf-Formstück 6 eingeschoben. Sie wird hierzu zunächst zusammengedrückt, das heisst die beiden Schenkel der V-Form werden ganz oder nahezu geschlossen. Dann lässt sich diese Zwischenplatte 19 ohne Weiteres zwischen die Formstücke 1,6 einschieben. Sie wird dann entspannt und spreizt sich elastisch in die hier gezeigte Form zurück, in welcher sie die beiden Formstücke 1,6 auf ihrer Hinterseite leicht voneinander wegspreizt und somit beabstandet. Jetzt können die beiden Luftkissen 13 zwischen die beiden Schenkel der Zwischenplatte 19 eingeschoben werden, sodass sie links und rechts zwischen den beiden Schenkeln 4,8 und 5,9 der beiden Formstücke 1,6 platziert sind. In dieser Ausgangsstellung ist der Kopf der zu therapierenden Person stabilisiert, jedoch noch nicht gegenüber der Schulter entlastet. Die Luftkissen 13 können jetzt durch Zusammendrücken des Balges 15 durch die zu therapierende Person aufgeblasen werden.

Dieses Aufblasen soll bewusst durch die betreffende Person selbst durchgeführt werden, denn diese kann durch das Pumpen ganz fein dosiert eine ihr angenehme oder gerade noch erträgliche Zugspannung auf ihre cervicale Wirbelsäule erzeugen. Umgekehrt kann sie diese Zuspannung bei Bedarf oder bei einem akuten Auftreten von Schmerz sofort wieder reduzieren oder beseitigen, indem sie das Einweg-Entlastungsventil 18 betätigt, wozu sie bloss auf den entsprechenden federbelasteten Druckknopf zu drücken braucht. Diese hier gezeigte Vorrichtung kann auch ohne die Zwischenplatte 19 eingesetzt werden. In diesem Fall werden die Luftkissen 13 direkt zwischen die hinteren Bereiche der Schenkel 4,8 und 5,9 der beiden Formstücke 1,6 eingelegt.

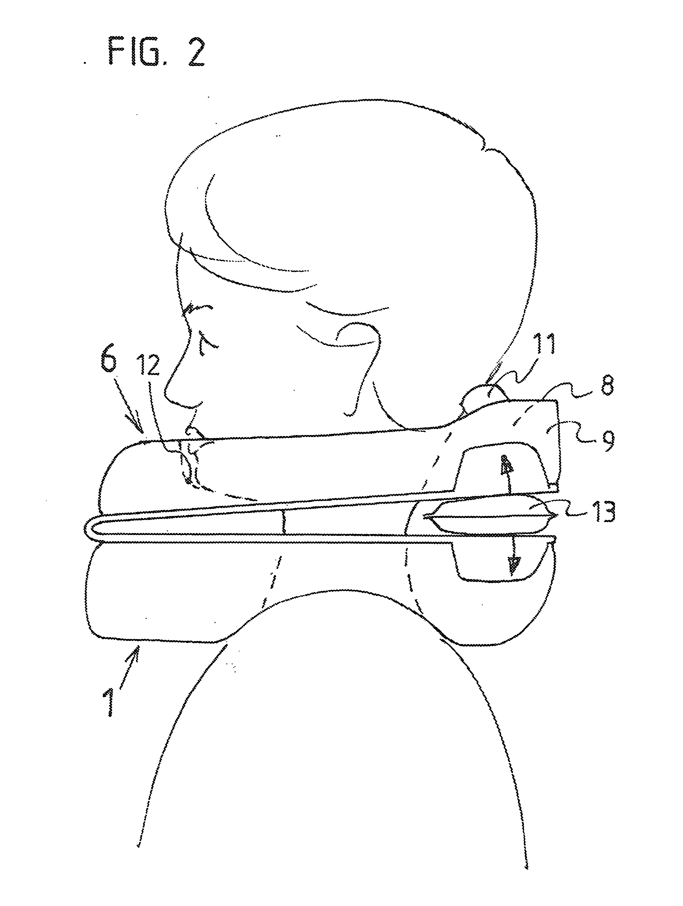

In Figur 2 sieht man die Vorrichtung an eine zu therapierende Person angelegt, von der Seite her gesehen. Das untere Schulter-Formstück 1 liegt auf der Schulter auf, während das obere Formstück 6, nämlich das Kopf-Formstück 6, den Kopf der Person an drei Punkten stützt, nämlich einerseits am Kinn durch die Ausnehmung 12, sowie am unteren hinteren Schädelrand durch die Höcker 11 auf der Oberseite der Schenkel 8,9 des Kopf-Formstückes 6. Zwischen diese beiden Formstücke 1,6 sind die Luftkissen 13 eingelegt. Es kann sich auch um ein einzelnes Luftkissen 13 handeln, das sich über die Breite der Vorrichtung erstreckt, also quer hinter dem Hals der Person verlaufend zwischen die beiden Schenkel der beiden Formstücke 1,6 eingelegt ist. Die Luftkissen 13 können mittels an ihnen angebrachter Klettbänder vor einem Verrutschen auf dem zum Beispiel unteren Formstück 1 gesichert sein. Wird der Balg 15 der zugehörigen Pumpvorrichtung betätigt, so werden die beiden Formstücke 1,6 mit ihren hinteren Enden wie mit den Pfeilen eingezeichnet voneinander weggespreizt, während sie vorne aufeinander aufliegen. Je nach Spreizlage wird die Wirbelsäule der therapierten Person im Nackenbereich mehr oder weniger entlastet.

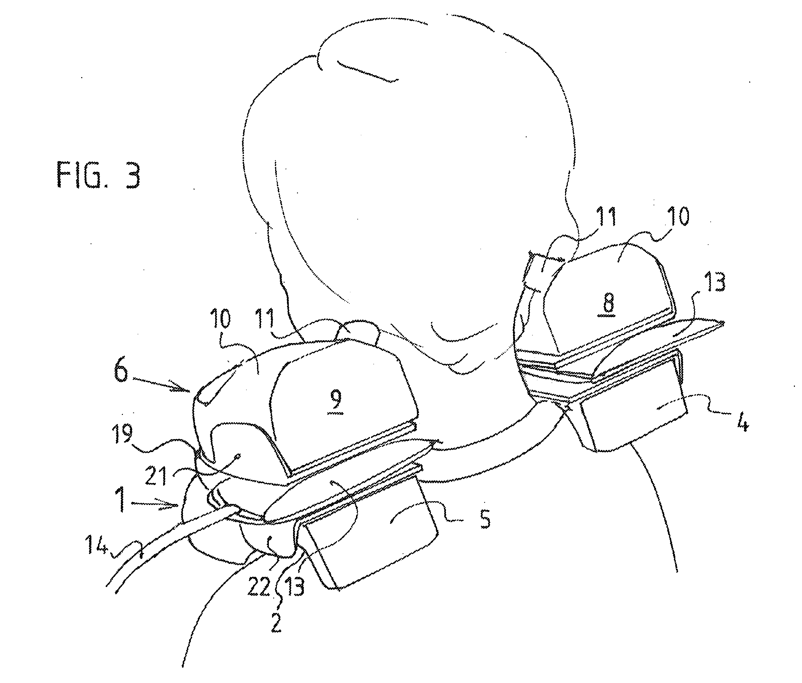

Die Figur 3 zeigt die Vorrichtung an eine zu therapierende Person angelegt, von schräg hinten gesehen. Man erkennt, dass die beiden Formstücke 1,6 auf der hinteren Seite offen sind, weil dort die beiden Schenkel 4,5 der Formstücke 1,6 enden. Aufgrund der Beschaffenheit des Materials der Formstücke 1,6 können deren Schenkel etwas auseinandergespreizt werden, was das Anlegen zunächst der Schulter-Formstückes 1 und hernach des Kopf-Schulterstückes 6 erleichtert. Die Ausbuchtung 2 für die Schulter der Person sichert den guten Pass-Sitz des Schulter-Formstückes 1 und beim Kopf-Formstück 6 ist die Dreipunktauflage von Bedeutung.

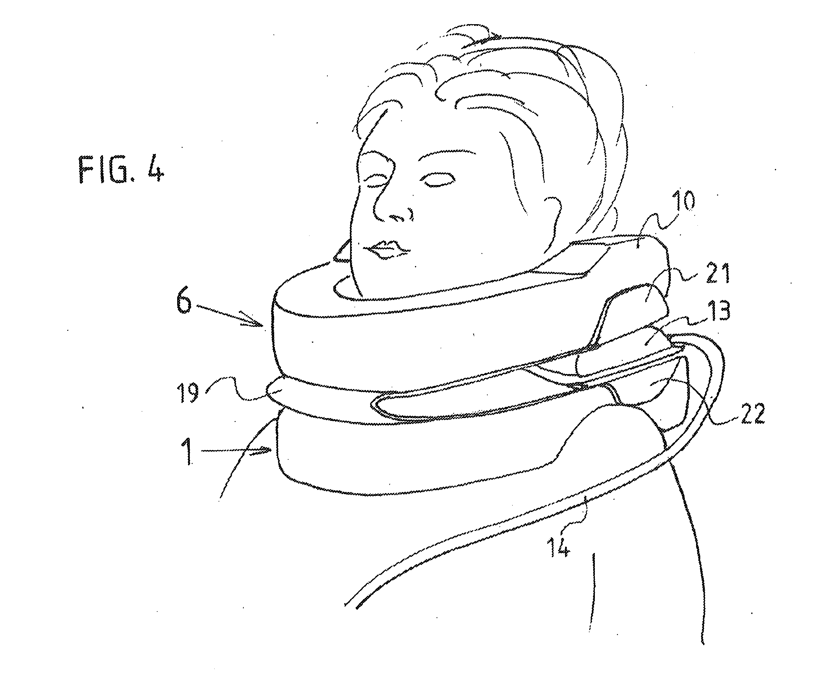

In Figur 4 ist die Vorrichtung an einer zu therapierende Person angelegt, von schräg vorne gesehen. Die Rundung auf der inneren Seite des Kopf-Formteils 6 schafft genügend Rum für die Wangen und den Hals. Bei nicht ganz schlanken Personen, solchen etwa mit einem ausgeprägten Doppelkinn oder mit gedrungenem Körperbau mit nur kurzem Hals wird dieser Raum besonders benötigt. Als besonders hilfreich und dem Wohlbefinden zuträglich ist ein periodisches Entlasten und wieder Belasten der cervicalen Wirbelsäule, wie das auch viele Physiotherapeuten bestätigen. Diese Therapie lässt sich mit dem hier vorgestellten Gerät äusserst wirksam und höchst einfach und bequem durchführen, und zwar von der zu therapierenden Person selbst.

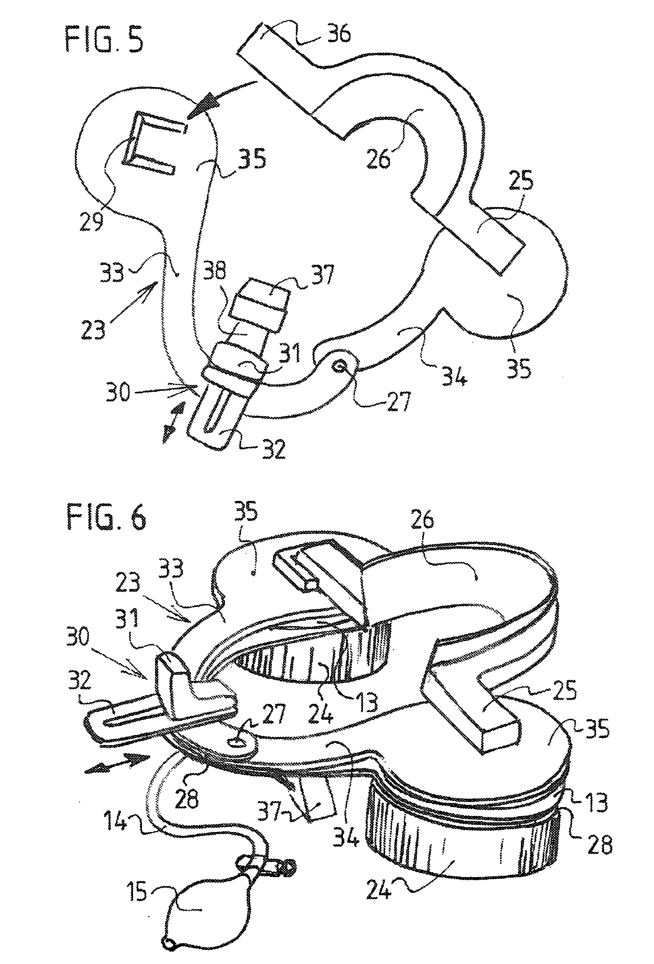

Die Figur 5 zeigt eine Vorrichtung gemäß der Erfindung Sie besteht aus einer kopfseitigen Formplatte 23, die zwei Schenkel 33,34 bildet, einen längeren Schenkel 33 und einen kürzeren Schenkel 34, die über ein Scharnier 27 zueinander verschwenkbar miteinander verbunden sind. An ihren Enden laufen diese Schenkel 33,34 in je eine hier kreisrunde Stützplatte 35 aus. Diese ganze Formplatte 23 kann aus einer Kunststoffplatte gefertigt sein, oder aus einer starken Schaumstoffplatte, die beschichtet sein kann, damit sie abwaschbar ist und somit gut reinigbar ist. Auf der einen kreisrunden Stützplatte 35, hier jener am Ende des Schenkels 34, ist ein Schliessriegel 25 mit seinem dortigen Ende fest befestigt. Er erstreckt sich zum gegenüberliegenden Schenkel 33 und ist in seiner Mitte in einen Bogen ausgeformt, sodass dessen Innenseite eine gepolsterte trichterförmige Stützfläche formt, von welcher somit eine Nackenstütze 26 gebildet ist. Wenn die beiden Schenkel 33,34 um das Scharnier 27 zueinander hin geschwenkt werden, längs des eingezeichneten gekrümmten Pfeiles, so kommt das andere, freie Ende 36 des Schliessriegels 25 schliesslich auf der kreisförmigen Stützplatte 35 des Schenkels 33 zu liegen. Auf dieser Stützplatte 35 sind Einrastrippen 29 angeformt. Wenn das freie Ende 26 des Schliessriegels 25 in diese Einrastrippen 29 einklickt, wird ein fester Schluss mit ihm gebildet. Die Vorrichtung bildet dann mit ihren zwei Schenkeln 33,34 nicht mehr bloss eine Hufeisenform, sondern einen festen und geschlossenen Ring. Durch diese Verschwenkbarkeit von zwei Schenkeln 33,34 ist die Vorrichtung sehr viel leichter um den Hals einer Person anlegbar. Mit offenen Schenkeln 33,34 wird sie angelegt und hernach werden die Schenkel 33,34 um den Hals der Person zueinander verschwenkt und der Schliessriegel 25 wird eingeklinkt, womit ein stabiler und fester Ring um den Hals/Schulterbereich der zu therapierenden Person gebildet ist. Die Nackenstütze 26 liegt dann satt am Nacken der Person an. Auf der gegenüberliegenden, vorderen Seite der Vorrichtung ist eine Kinnstütze 30 am Schenkel 33 angebaut. Diese sitzt auf einer längs der eingezeichneten Pfeile verschiebbaren Verstellplatte 32, die ein nach oben ragendes, weichelastisches Kinnpolster 31 trägt. Die Kinnstütze 30 wird an das Kinn der zu therapierenden Person angepasst und dann in der richtigen Lage gesichert. Dann sitzt das Kinnpolster 31 satt am Kinn der Person und stützt es. Unten am Schenkel 33, unterhalb der Kinnstütze 30, ist eine Brust-Stützpolster 37 vorhanden, welches von einem in der Länge verstellbaren Bügel 38 gehalten ist, der unten am Schenkel 33 befestigt ist. Dieser Bügel 38 lässt sich soweit verstellen, bis das endseitige Brust-Stützpolster 37 am Brustbein der zu therapierenden Person anliegt und die Vorrichtung gegenüber der Brust abstützt.

In Figur 6 ist diese Vorrichtung in Anwendungslage gezeigt, also mit geschlossenen Schenkeln 33,34. Auf der Unterseite der Formplatte 30 ist eine elastische Kunststoff-Platte 28 befestigt, jedoch nur im Bereich der gebogenen Schenkel 33,34. Gegen die endseitigen Stützplatten 35 hin ist die Kunststoff-Platte 28 lose und kann daher gegenüber den Stützplatte 35 leicht nach unten gebogen werden. Auf der Oberseite dieser Kunststoffplatte und unterhalb der Stützeplatte n35 ist je ein Luftkissen 13 befestigt, das ohne Luft etwa die gleiche Grundform aufweist wie die darüberliegende Stützplatte 35. Es wird über einen Schlauch 14 mit Luft versorgt. Dieser Schlauch 14 ist einseitig mit einem Gummibalg 15 ausgerüstet. Durch Zusammendrücken wird Luft in die Luftkissen 13 gepumpt und die Kunststoffplatte 28 wird von den Stützplatte 35 weggespreizt. Auf der Unterseite der Kunststoffplatte 28, unterhalb der Stützplatten 35, sind hier zylinderförmige Schaumstoffpolster 24 befestigt, zum Beispiel mittels Klettverschlüssen oder Druckknöpfen. Diese Schaumstoffpolster 24 kommen in Anwendungslage auf den Schultern der zu therapierenden Person zu liegen. Sie können durch mehr oder weniger hohe Polster ausgetauscht werden, sodass bei jedem Patienten eine optimale Passlage der Vorrichtung erzielbar ist. Die Vorrichtung ist dann gut an eine Person angelegt, wenn ihr Nacken satt von der Nackenstütze 26 gestützt wird, das Kinn satt auf dem Kinnpolster 31 aufliegt und die Vorrichtung mit dem Brustpolster 37 auf dem Brustbein abgestützt ist. Durch Aufpumpen der Luftkissen wird sodann der Nacken durch eine Abstützung auf den beiden Schultern sanft angehoben.

Es ist klar, dass die Vorrichtung und ihre Funktion auch durch abgewandelte Konstruktionen realisierbar ist. Hauptsache ist, dass eine Schulterauflage und darauf anhebbare Kopfauflage für den Nacken- und Kinnbereich vorhanden sind, sowie ein Hebemittel 13 zwischen dem hinteren Bereich der Schulterauflage und der Kopfauflage.

## Patentansprüche

1. Vorrichtung zum Entlasten und Therapieren der cervicalen Wirbelsäule, bestehend aus einer Schulterauflage und darauf anhebbaren Kopfauflage für den Nacken- und Kinnbereich, sowie einem pneumatischen Hebemittel (13) mit Luftkissen zwischen dem hinteren Bereich der Schulterauflage und der Kopfauflage und zugehörigem Gummibalg (15) zum Pumpen, und mit Entlastungs-Einwegventil und Manometer (16) im Zufuhrschlauch (14) zum Luftkissen (13) zur Messung und Anzeige des Innendruckes in den Luftkissen,
***dadurch gekennzeichnet*,**
**dass** die Kopfauflage von einer Formplatte (23) aus zwei gegeneinander verschwenkbaren Schenkeln (33,34) mit endseitigen Stützplatten (35) besteht, die miteinander kraftschlüssig verbindbar sind, und dass die Schulterauflage durch zwei Kunststoffplatten (28) gebildet ist, die an der Unterseite der Schenkel (33,34) befestigt sind und von denselben wegdehnbar oder scharnierend wegschwenkbar sind und auf ihrer Oberseite, unterhalb der Stützplatten (35), je ein Luftkissen (13) tragen, und auf ihrer Unterseite, unterhalb der Luftkissen (13), mit lösbar befestigten Schulter-Stützpolstern (24) ausgerüstet sind.

2. Vorrichtung zum Entlasten und Therapieren der cervicalen Wirbelsäule nach Anspruch 1, ***dadurch gekennzeichnet*,**
**dass** die Schulterauflage auf der Unterseite der Schenkel (33,34) so an denselben befestigt ist, dass ein Schwenkscharnier gebildet ist, sodass die Schenkel (33,34) gegenüber der Schulterauflage um eine etwa in der Ebene der Schulterauflage verlaufende Schwenkachse von derselben aufschwenkbar sind.

3. Vorrichtung zum Entlasten und Therapieren der cervicalen Wirbelsäule, nach einem der vorangehenden Ansprüche, ***dadurch gekennzeichnet*,**
**dass** die zwei gegeneinander verschwenkbaren Schenkel (33,34) über einen auf dem einen Schenkel (34) befestigten Schliessriegel (25) miteinander kraftschlüssig verbindbar sind.

4. Vorrichtung zum Entlasten und Therapieren der cervicalen Wirbelsäule, nach einem der vorangehenden Ansprüche, ***dadurch gekennzeichnet*,**
**dass** die zwei gegeneinander verschwenkbaren Schenkel (33,34) über einen auf dem einen Schenkel (34) befestigten Schliessriegel (25) miteinander kraftschlüssig verbindbar sind, welcher im geschlossenen Zustand der Schenkel (33,34) eine Nackenstütze (26) bildet.

5. Vorrichtung zum Entlasten und Therapieren der cervicalen Wirbelsäule nach einem der vorangehenden Ansprüche, ***dadurch gekennzeichnet*,**
**dass** am einen Schenkel (33) auf seiner Oberseite ein verstellbares Kinnpolster (31) befestigt ist, und auf seiner Unterseite ein in der Höhenlage verstellbares Brust-Stützpolster (37).

6. Vorrichtung zum Entlasten und Therapieren der cervicalen Wirbelsäule nach einem der vorangehenden Ansprüche, ***dadurch gekennzeichnet*,**
**dass** die Kopfauflage im geschlossenen Zustand der Schenkel (22,23) und eingerastetem Schliessriegel (25) eine Auflagefläche für den hinteren unteren Schädelrand bildet, sowie das Kinnpolster (31) eine Auflagefläche für das Kinn, zur Bildung einer stabilen und definierten Mehrpunktauflage des Kopfes.

## Claims

1. Device for relieving and treating the cervical spine, consisting of a shoulder pad and head support for the neck and chin aera, as well as a pneumatic lifting means (13) with air cushions between the back of the shoulder pad and the head support, and a pertaining bellows (15) for pumping, and a discharge one-way-valve and pressure gauge (16) in the supply hose (14) for the air cushions (13) for measuring and displaying of the internal pressure in the air cushions, **characterized in that** the head support consists of a form plate (23) of two branches (33,34), swivel-mounted toward each other, with support plates (35) at their ends, and which are force-fittingly connectable with each other, and that the shoulder rest consists of two plastic plates (28) attached on the lower side of the branches (33,34) and which are ductible away from them or swivel away from them by a hinge, and which bear, on their upper side, below the support plates (35), an air cushion (13) each, and which are equiped with detachable shoulder pads (24) on their lower side, below the air cushions (13).

2. Device for relieving and treating the cervical spine according to claim 1, **characterized in that** the shoulder rest on the lower side of the branches (33,34) is attached in this manner on them, that a hinge is formed, so that the branches (33,34) are swivel-mounted upwards from the shoulder rest around an axis which is approximately extending in the plane of the shoulder rest.

3. Device for relieving and treating the cervical spine according to claim 1, **characterized in that** the two branches (33,34) which are swivel-mounted toward each other are force-fittingly interlockable with each other by an interlock (25) mounted on one of the branches (34).

4. Device for relieving and treating the cervical spine according to claim 1, **characterized in that** the two branches (33,34) which are swivel-mounted toward each other are force-fittingly interlockable with each other by an interlock (25) mounted on one of the branches (34), and said interlock (25) forms a neck support (26) when the branches (33,34) are in their closed state.

5. Device for relieving and treating the cervical spine according to claim 1, **characterized in that** on one of the branches (33) on its upper side, an adjustable chin pad (31) is mounted, and on its lower side a breast support pad (37) which is adjustable in height.

6. Device for relieving and treating the cervical spine according to claim 1, **characterized in that** the head support forms a support surface for the rear lower edge of the skull when the branches (33,34) are in their closed state and the interlock (25) is locked, and that the chin pad (31) provides a support surface for the chin, for building a stable and definite multi-point-support for the head.

## Revendications

1. Dispositif pour soulager et soigner la colonne vertébrale cervicale composé d'une surface d'appui pour l'épaule et d'une surface d'appui pour la tête susceptible d'être soulevée pour la zone du cou et du menton et composé également de moyens élévateurs (13) pneumatiques avec un coussin d'air situé entre la zone arrière de la surface d'appui pour l'épaule et la surface d'appui pour la tête ainsi que le ballon en caoutchouc (15) associé pour pomper, et **caractérisé en ce que**,
la surface d'appui de la tête est composée d'une plaque de formage (23) composée de deux branches (33, 34), susceptibles d'être pivotées l'une par rapport à l'autre, avec des plaque d'appui (35) terminales, lesquelles plaques sont susceptibles d'être liées l'une par rapport à l'autre par liaison de force, et **en ce que** la surface d'appui pour l'épaule est formée par deux plaques en matière plastique (28) qui sont fixées sur le côté inférieur des branches (33, 34) et qui sont susceptibles d'être écartées desdites branches ou susceptibles d'être pivoté en l'écartant comme une charnière desdites branches et lesquelles plaques en matière plastique portent sur leur côté supérieur en-dessous des plaques d'appui (35) à chaque fois un coussin d'air et lesquelles plaques sont équipées sur leur côté inférieur situé en-dessous des coussins d'air (13) d'épaulettes de protection (24) fixées de façon mobile.

2. Dispositif pour soulager et soigner la colonne vertébrale cervicale selon la revendication 1, **caractérisé en ce que**, la surface d'appui pour l'épaule est fixée sur le côté inférieur des branches (33, 34) de manière à ce qu'une charnière de pivotement, de manière à ce que les branches (33, 34) puissent être pivotées par rapport à la surface d'appui pour l'épaule autour d'un axe de pivotement s'étendant à peu près dans le plan de la surface d'appui pour l'épaule.

3. Dispositif pour soulager et soigner la colonne vertébrale cervicale selon une des revendications précédentes, **caractérisé en ce que** les deux branches (33, 34), susceptibles d'être pivotées l'une par rapport à l'autre, peuvent être liées l'une à l'autre de façon par liaison de force via un verrou de fermeture (25) fixée à ladite branche (34).

4. Dispositif pour soulager et soigner la colonne vertébrale cervicale selon une des revendications précédentes, **caractérisé en ce que** les deux branches (33, 34), susceptibles d'être pivotées l'une par rapport à l'autre, peuvent être liées l'une à l'autre de façon par liaison de force via un verrou de fermeture (25) fixée à ladite branche (34), lequel verrou de fermeture (25) forme dans un état de fermeture des branches (33, 34) un appui pour le cou (26).

5. Dispositif pour soulager et soigner la colonne vertébrale cervicale selon une des revendications précédentes, **caractérisé en ce qu'**un coussin pour le menton (31) réglable est fixé sur une branche (33) sur le côté supérieur de ladite branche, et **en ce qu'**un coussin de protection pour la poitrine (37) réglable en hauteur est fixé sur le côté inférieur de ladite branche.

6. Dispositif pour soulager et soigner la colonne vertébrale cervicale selon une des revendications précédentes, **caractérisé en ce que** la surface d'appui pour la tête forme dans l'état de fermeture des branches (22, 23) et lorsque le verrou de fermeture (25) est encliqueté, une surface d'appui pour le bord du crâne arrière inférieur et le coussin pour le menton (31) forme une surface d'appui pour le menton afin de réaliser un support multipoint stable et défini pour la tête.
